# EUROPEAN PATENT APPLICATION

(11) **EP 3 632 479 A1**
(43) Date of publication of application: **08.04.2020**
(21) Application number: 18020481.0
(22) Date of filing: 04.10.2018
(51) Int. Cl.: A61L 27/06, A61C 8/00, A61L 27/30, A61L 27/32

(54) **NANOPOROUS COATING OF TITANIUM DIOXIDE ON TITANIUM ALLOY AND THE METHOD OF MANUFACTURING**

(71) Applicant: Nano-implant Sp. z o.o., 87-100 Torun (PL)
(72) Inventor: Radtke, Aleksandra, 87-100 Toru? (PL); Piszczek, Piotr, 87-100 Toru? (PL)
(74) Representative: Cwiklinski, Grzegorz

(57) **Abstract**

The method of producing a nanoporous coating with nanotooth morphology, enriched with a nanometre layer of hydroxyapatite, on the surface of the Ti6Al4V titanium alloy implant, characterised in that the method consists in degreasing the surface of the implant by immersion for 2 to 8 minutes in acetone using an ultrasonic cleaner, followed by immersion in ethyl alcohol for 2 to 8 minutes and rinsing for 2 to 8 minutes in distilled water using an ultrasonic cleaner for 2 to 8 minutes; and once removed from the ultrasound cleaner, the titanium alloy implant is immersed in a mixture of hydrofluoric acid, nitric(V) acid and distilled water, mixed in a volume ratio of 1:4:5, for 20 to 40 seconds, during the pulsed anodic oxidation of the surface of the titanium alloy implant in a solution of ethylene glycol containing 0.3 to 2% by weight of water and 0.2 to 0.4% hydrofluoric acid, wherein the process is performed in two steps: first for 60 minutes at 30 V and 10 V by changing one voltage value to the other every 5 minutes, and then by carrying out six cycles at 30 V and without voltage (0 V), changing one value to the other so that the process at 30 V lasts 50 seconds and the process without voltage lasts 10 minutes, followed by rinsing in distilled water with Al₂O₃ powder in the ultrasonic cleaner; wherein, on the produced titanium dioxide layers featuring the morphology of nanoteeth, with a thickness of approx. 100 nm, with the 300-600 nm dia. pores visible under the electron microscope, a hydroxyapatite layer is applied in a two-stage process: first, a thin layer of calcium carbonate is produced using the Atomic Layer Deposition (ALD) method, using the Ca(thd) as the precursor, where thd means 2,2,6,6-tetramethyl-3,5-heptanedione, in the presence of O₃, with the precursor evaporation temperature of 180-195°C, with a deposition temperature of 240-260°C, with the number of cycles 100 to 500 (deposition programme: pulse Ca(thd)₂ - 1 second, pulse N₂ - 1 second, pulse O₃ - 1 second, and pulse N₂ - 1 second); and the CaCO₃ nanolayer produced is converted into a nanocrystalline layer of hydroxyapatite by placing in 0.2 M alkaline diammonium hydrogen phosphate in 1 M NH₃ at 95°C for 0.5 to 2 minutes.

## Description

This invention relates to a nanoporous coating on the surface of titanium alloy, particularly on the surface of Ti6Al4V titanium alloy and the method of manufacturing with a strictly defined structure and morphology, for the production of 3D implants used in orthopaedics, maxillo-facial surgery and dentistry, for the best possible osteointegration, i.e. the process of connection between the implant and a patient's bones.

Implants made of Ti6Al4V titanium alloy for orthopaedics, maxillo-facial surgery and dentistry in comparison with other metallic materials feature very good biocompatibility and, in addition, good corrosion resistance, the lowest Young's module, high relative strength and low specific weight.

There are known methods of surface modification of metallic biomaterials, including Ti6Al4V titanium alloy. These methods are mechanical (sandblasting, machining, polishing) and physical (physical deposition from the gaseous phase, ion-assisted deposition, evaporation, plasma discharge). Their main objective is to obtain a specific topography and roughness of the implant surface, i.e. the surface of better adhesion to the bone.

There are also known chemical methods of modification of the substrate of metallic biomaterial, such as Ti6Al4V alloy. These methods may include chemical vapour deposition, electrochemical oxidation, sol-gel method as well as chemical reactions in acidic, alkaline or hydrogen peroxide environments. All of these methods lead to the formation of a layer of titanium dioxide on the surface of the implant, featuring varied structure and morphology that provide the original surface of the Ti6Al4V alloy with better biocompatibility and bioactivity.

There is also a known method of an electrochemical oxidation of the substrate of metallic biomaterial, such as the Ti6Al4V alloy. This method is used to obtain a layer of nanotubes of titanium dioxide (TiO₂) with controlled morphology. The diameter of the TiO₂ nanotubes is a derivative of the voltage applied during the oxidation process and their length (or layer thickness) depends on the duration of the electrochemical oxidation process.

There are known various conditions for the process of oxidation of metallic substrate, such as the Ti6Al4V alloy, meaning the process voltage ranges from 1 V to 150 V, the process lasts for a few minutes to several hours, type of electrolyte (aqueous or anhydrous), concentration of fluoride ions directly attributable for the formation of the TiO₂ nanotubes ranging from 0.05 M to 0.5 M. There are also known modifications of the oxidation process of titanium alloy substrate through the use of variable voltage to form the so-called bamboo nanotubes. These nanotubes are interconnected by crosswise elements, as is the case in bamboo shoots, with a smooth inside. They are created using a relatively high voltage, for example 120 V, alternately with a lower voltage (40 V). This process is performed in the presence of anhydrous electrolyte - ethylene glycol. High voltage creates favourable conditions for the formation of smooth well-separated nanotubes, while lowering the potential creates favourable conditions for growth, the inhibition of nanotubes growth and the formation of nodules around the base of the formed nanotubes. This stage must be sufficiently long, at least 30 minutes, to be able to observe the successful formation of connections. Following a voltage jump, the production of smooth structures continues.

All the above parameters (potential value, process duration, electrolyte, fixed/variable potential) influence the structure, morphology and biological properties of the resulting layers of titanium dioxide nanotubes.

There are known methods of modification of the titanium dioxide layer with hydroxyapatite in order to increase biointegration activity of the system. These methods may include thermal deposition, an inexpensive method used to achieve a high rate of 30-200 µm thick layer deposition. The disadvantages of this method are the high temperature of the process, as it can cause decomposition, and rapid cooling that results in the formation of an amorphous coating. Another method is ion deposition that is suitable to obtain a compact coating of uniform thickness within the range of 0.5 - 3 µm. Unfortunately, this method can only be used to obtain amorphous coatings; its high cost and time-consuming nature are also a disadvantage. Obtaining compact porous coatings as well as amorphous and crystalline coatings with a layer thickness of 0.05-5 µm is made possible by laser ablation. a method that can be used to obtain hydroxyapatite layers at a high speed, with a uniform thickness of 0.1-2.0 mm on substrates of irregular shape and morphology, is electrophoretic deposition. However, the sol-gel method is most often used due to a number of advantages: low process temperature, relatively low costs and the possibility of obtaining coatings with a thickness of less than 1 µm. The microstructure of coatings can be shaped by the selection of chemical parameters or process conditions. However, all the above mentioned methods lead to the formation of hydroxyapatite layers characterised by high brittleness and relatively weak adhesion to the surface, i.e. they are easily detached. In turn, this is the cause for which their applicability loses its value, although biocompatibility is exceptionally good.

The inconvenience of using implants made of the Ti6Al4V titanium alloy is that there are cases of rejection of the implant by the recipient's body, despite the relatively high biocompatibility of titanium alloy, and inflammatory conditions after the implantation process require antibiotic treatment. Moreover, there is a group of patients who, due to their age or disease, are excluded from the group of people who undergo the implant process, as they are particularly susceptible to bacterial infections leading to local inflammatory conditions. These are children, people over 60 years of age, diabetics, people with diseases of the immune system and people with addictions. For this reason, methods are being sought to modify the surface of the titanium alloy in order to provide it with additional properties - better osteointegration and antibacterial action.

The inconvenience of using mechanical and physical modification of the surface of metallic biomaterials, including the Ti6Al4V titanium alloy, is that they fail to improve the anti-inflammatory and antibacterial properties of their surfaces, despite the increased porosity of the coating to improve the osteointegration properties of the implant.

The inconvenience of using chemical and electrochemical modification of the surface of metallic biomaterials, including the Ti6Al4V titanium alloy, is that they fail to have an effect on the modified anti-inflammatory and antibacterial properties of the implant, despite the possibility of controlling the surface morphology of the coating improving the osteointegration properties of the implant.

The inconvenience of using the sol-gel method for the enrichment of the TiO₂ layer with hydroxyapatite grains lies with the limited possibility of obtaining a permanent connection between TiO₂ and hydroxyapatite, and hence the production of systems with low application potential for implantology.

The essence of the invention is that the nanoporous coating of titanium dioxide, enriched with a layer of hydroxyapatite on the surface of titanium alloy, has a morphology of nanoteeth.

The method of producing a nanoporous coating with nanotooth morphology, enriched with a nanometre layer of hydroxyapatite, on the surface of the Ti6Al4V titanium alloy implant consists in degreasing the surface of the implant by immersion for 2 to 8 minutes in acetone using an ultrasonic cleaner, followed by immersion in ethyl alcohol for 2 to 8 minutes and rinsing for 2 to 8 minutes in distilled water using an ultrasonic cleaner for 2 to 8 minutes. Once removed from the ultrasound cleaner, the titanium alloy implant is immersed in a mixture of hydrofluoric acid, nitric(V) acid and distilled water, mixed in a volume ratio of 1:4:5, for 20 to 40 seconds, during pulsed anodic oxidation of the surface of the titanium alloy implant in a solution of ethylene glycol containing 0.3 to 2% by weight of water and 0.2 to 0.4% hydrofluoric acid, wherein the process is performed in two steps: first for 60 minutes at 30 V and 10 V by changing one voltage value to the other every 5 minutes, and then by carrying out six cycles at 30 V and without voltage (0 V), changing one value to the other so that the process at 30 V lasts 50 seconds and the process without voltage lasts 10 minutes, followed by rinsing in distilled water with Al₂O₃ powder in the ultrasonic cleaner.

On the produced titanium dioxide layers featuring the morphology of nanoteeth, with a thickness of about 100 nm, with the 300-600 nm dia. pores visible under the electron microscope, a hydroxyapatite layer is applied in a two-stage process: first, a thin layer of calcium carbonate is produced using the Atomic Layer Deposition (ALD) method, using the Ca(thd) as the precursor, where thd means 2,2,6,6-tetramethyl-3,5-heptanedione, in the presence of O₃, with the precursor evaporation temperature of 185-195°C, with the deposition temperature of 240-260°C, with the number of cycles being 500 (deposition programme: pulse Ca(thd)₂ - 1 second, pulse N₂ - 1 second, pulse O₃ - 1 second, and pulse N₂ - 1 second); and the CaCO₃ nanolayer produced is converted into a nanocrystalline layer of hydroxyapatite by placing in 0.2 M alkaline diammonium hydrogen phosphate in 1 M NH₃ at 95°C for 0.5 to 2 minutes.

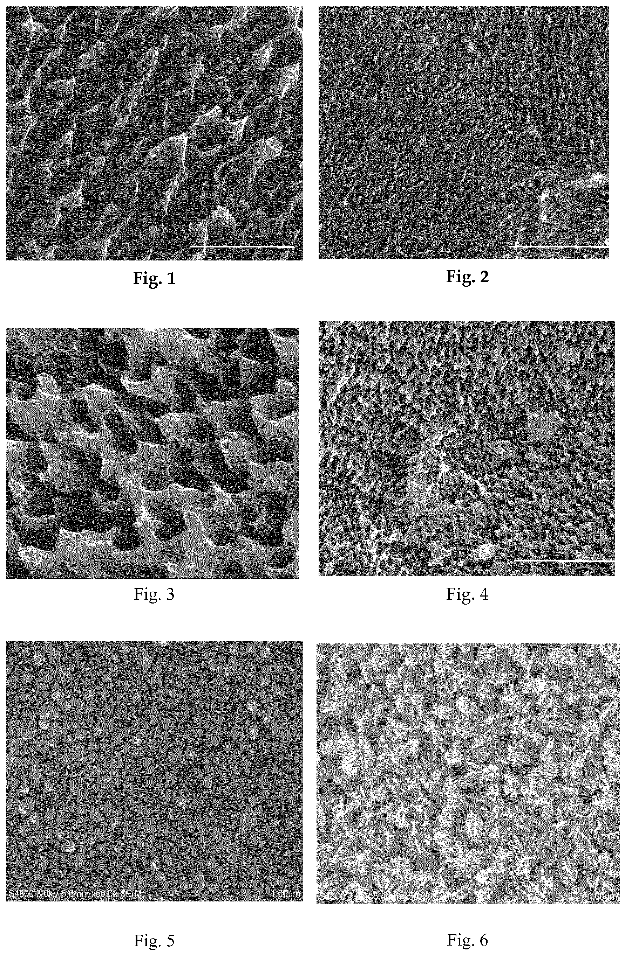

The structure of the obtained titanium dioxide nanolayers is shown on Figs. 1, Fig. 2, Fig. 2 and Fig. 4, coated with CaCO₃ in Fig. 5, with hydroxyapatite in Fig. 6.

The object of the invention is further illustrated by embodiments, without limiting the scope of invention protection.
Embodiment One. a method of producing nanoporous coating with nanotooth morphology, enriched with a 200 nanometre layer of hydroxyapatite, on the surface of the substrate being an implant made of the grade 5 Ti6Al4V titanium alloy that contain 90% titanium, 6% aluminium and 4% vanadium.
   Stage 1. The titanium alloy substrate was degreased by immersion in acetone for 5 minutes using an ultrasonic cleaner, then immersion in ethanol for 5 minutes, and rinsed for 5 minutes in distilled water using the ultrasonic cleaner again. Once removed from the ultrasonic cleaner, the titanium alloy substrate was immersed in a mixture of hydrofluoric acid, nitric(V) acid and distilled water (mixed in a volume ratio of 1:4:5) for 30 seconds.
   Stage 2. The process of the pulse anodic oxidation of the surface of the titanium alloy substrate was carried out in two steps: first at 30 V and 10 V for 60 minutes, by changing one voltage to the other every 5 minutes, followed by six cycles at 30 V and without voltage (0 V), by changing one voltage to the other so that the process at 30 V lasted for 50 seconds and the process without voltage lasted for 10 minutes. The procedure takes place in the presence of ethylene glycol solution containing 0.2 M hydrofluoric acid and water in the amount of 0.3-2% by weight. After the anodic oxidation process, the surface of the titanium alloy substrate is rinsed in distilled water in an ultrasonic bath with the addition of Al₂O₃ powder. The whole of the operations associated with the pulse anodic oxidation process leads to the formation of a 100 nm thick titanium dioxide nanotooth layer on the titanium alloy substrate, with 300-600 nm dia. pores visible under the electron microscope.
   Stage 3. a two-stage process is used to apply a hydroxyapatite layer on the titanium dioxide layer: first, a thin layer of calcium carbonate is produced using the Atomic Layer Deposition (ALD) method, and then the produced CaCO₃ is converted into crystalline hydroxyapatite, using Ca(thd)₂ and O₃ as the precursor, whereas the process temperatures amounted to 188°C as the evaporation temperature of the precursor and 250°C as the deposition temperature, with the number of cycles of 500 (deposition programme: pulse Ca(thd)₂ - 1 second, pulse N₂ - 1 second, pulse O₃ - 1 second, and pulse N₂ - 1 second). The conversion of CaCO₃ into nanocrystalline hydroxyapatite is carried out by wet synthesis, by placing the implant in an environment of 0.2 M of alkaline diammonium hydrogen phosphate in 1 M NH₃ at 95°C.
Embodiment Two. a method of producing nanoporous coating with nanotooth morphology, enriched with a 40 nanometre layer of hydroxyapatite, on the surface of the substrate being an implant made of the grade 5 Ti6Al4V titanium alloy that contain 90% titanium, 6% aluminium and 4% vanadium.
   Stage 1. The titanium alloy substrate was degreased by immersion in acetone for 5 minutes using an ultrasonic cleaner, then immersion in ethanol for 5 minutes, and rinsed for 5 minutes in distilled water using the ultrasonic cleaner again. Once removed from the ultrasonic cleaner, the titanium alloy substrate was immersed in a mixture of hydrofluoric acid, nitric(V) acid and distilled water (mixed in a volume ratio of 1:4:5) for 30 seconds.
   Stage 2. The process of pulse anodic oxidation of the surface of the titanium alloy substrate was carried out in two steps: first at 30 V and 10 V for 60 minutes, by changing one voltage to the other every 5 minutes, followed by six cycles at 30 V and without voltage (0 V), by changing one voltage to the other so that the process at 30 V lasted for 50 seconds and the process without voltage lasted for 10 minutes. The process is carried out in ethylene glycol solution containing water in the amount of 0.3-2% by weight and hydrofluoric acid in the amount of 0.3%. After the anodic oxidation process, the surface of the titanium alloy substrate is rinsed in distilled water in the ultrasonic cleaner with the addition of Al₂O₃ powder. a layer of titanium dioxide in the form of nanoteeth, 100 nm thick, with 300-600 nm dia. pores visible under the electron microscope, is produced on the surface of the titanium alloy.
   Stage 3. a two-stage process is used to apply a hydroxyapatite layer on the titanium dioxide layer: first, a thin layer of calcium carbonate is produced using the Atomic Layer Deposition (ALD) method, and then the produced CaCO₃ is converted into crystalline hydroxyapatite, using Ca(thd)₂ and O₃ as the precursor, whereas the process temperatures amounted to 188°C as the evaporation temperature of the precursor and 250°C as the deposition temperature, with the number of cycles of 100 (deposition programme for one cycle: pulse Ca(thd)₂ - 1 second, pulse N₂ - 1 second, pulse O₃ - 1 second, and pulse N₂ - 1 second). The conversion of CaCO₃ into nanocrystalline hydroxyapatite is carried out by wet synthesis, by placing the implant in an environment of 0.2 M of alkaline diammonium hydrogen phosphate in 1 M NH₃ at 95°C for 1 minute.

The testing of adhesion and proliferation of fibroblast and osteoblast cells on the surface of TiO₂ nanotooth layers, enriched with hydroxyapatite (TiO₂/HA), produced on the surface of the implant, was conducted with the use of the fibroblast cell line L929 and the osteoblast cell line M63. The obtained coatings showed 2-fold higher cell adhesion after 24 hours with the presence of the TiO₂ nanoteeth enriched with 200 nm HA and 40 nm HA on the surface, as compared to the reference sample, i.e. the titanium alloy implant without any TiO₂ coating. The testing of proliferation (growth) of fibroblast and osteoblast cells after 72 hours showed four times more fibroblasts in the presence of about 100 nm by mass silver nanograins on the surface of the TiO₂ nanotube layer and 6 times more cells in the presence of about 40 nm of the HA layer, as compared to the reference sample, i.e. the titanium alloy implant without TiO₂ nanotube coating with silver. For the osteoblasts, the above values were 3 and 4, respectively.

Microbiological examination of the nanotooth layers enriched with HA (hydroxyapatite)TiO₂/HA, produced on the surface of the implant, was performed with the use of Staphylococcus aureus ATCC 29213 and Staphylococcus aureus H9 bacteria. The produced coatings inhibited the development of biofilm by about 50% for Staphylococcus aureus ATCC 29213 and 65% for Staphylococcus aureus H9 bacteria, with the presence of about 200 nm HA on the surface of the TiO₂ nanoteeth layer. With the presence of about 45 nm HA layer on the surface of the TiO₂ nanoteeth, the biofilm development was inhibited by about 55% for Staphylococcus aureus ATCC 29213 and 69% for Staphylococcus aureus H9 bacteria. The microbiological examination was performed compared to the reference sample, i.e. the Ti6Al4V titanium alloy 3D implant without any TiO₂ nanotooth layer enriched with hydroxyapatite (TiO₂/HA).

The tests performed showed that the obtained layer of titanium dioxide with hydroxyapatite featuring the structure and morphology of nanoteeth significantly improves the osteointegration process (the process of connection between the implant and a patient's bone) and provides the 3D implant with antimicrobial properties.

## Claims

1. A nanoporous coating of titanium dioxide on the surface of titanium alloy, particularly on the surface of a 3D made of the Ti6Al4V titanium alloy, with a thickness of about 100 nm, featuring the morphology of nanoteeth, enriched with hydroxyapatite nanograins.

2. The method of producing a nanoporous coating with nanotooth morphology, enriched with a nanometre layer of hydroxyapatite, on the surface of the Ti6Al4V titanium alloy implant, **characterised in that** the method consists in degreasing the surface of the implant by immersion for 2 to 8 minutes in acetone using an ultrasonic cleaner, followed by immersion in ethyl alcohol for 2 to 8 minutes and rinsing for 2 to 8 minutes in distilled water using an ultrasonic cleaner for 2 to 8 minutes; and once removed from the ultrasound cleaner, the titanium alloy implant is immersed in a mixture of hydrofluoric acid, nitric(V) acid and distilled water, mixed in a volume ratio of 1:4:5, for 20 to 40 seconds, during the pulsed anodic oxidation of the surface of the titanium alloy implant in a solution of ethylene glycol containing 0.3 to 2% by weight of water and 0.2 to 0.4% hydrofluoric acid, wherein the process is performed in two steps: first for 60 minutes at 30 V and 10 V by changing one voltage value to the other every 5 minutes, and then by carrying out six cycles at 30 V and without voltage (0 V), changing one value to the other so that the process at 30 V lasts 50 seconds and the process without voltage lasts 10 minutes, followed by rinsing in distilled water with Al₂O₃ powder in the ultrasonic cleaner; wherein, on the produced titanium dioxide layers featuring the morphology of nanoteeth, with a thickness of approx. 100 nm, with the 300-600 nm dia. pores visible under the electron microscope, a hydroxyapatite layer is applied in a two-stage process: first, a thin layer of calcium carbonate is produced using the Atomic Layer Deposition (ALD) method, using the Ca(thd) as the precursor, where thd means 2,2,6,6-tetramethyl-3,5-heptanedione, in the presence of O₃, with the precursor evaporation temperature of 180-195°C, with a deposition temperature of 240-260°C, with the number of cycles 100 to 500 (deposition programme: pulse Ca(thd)₂ - 1 second, pulse N₂ - 1 second, pulse O₃ - 1 second, and pulse N₂ - 1 second); and the CaCO₃ nanolayer produced is converted into a nanocrystalline layer of hydroxyapatite by placing in 0.2 M alkaline diammonium hydrogen phosphate in 1 M NH₃ at 95°C for 0.5 to 2 minutes.

3. The method according to claim 2, **characterised in that** the degreasing of the 3D implant takes place with the use of an ultrasonic cleaner and acetone (pure for analysis grade), ethyl alcohol (pure for analysis grade) and distilled water.

4. The method according to claim 2, **characterised in that** the activation of the implant surface consists in a 30-second immersion of the implant in a mixture of hydrofluoric acid (pure for analysis grade), nitric(V) acid (pure for analysis grade) and distilled water, mixed in a volume ratio of 1:4:5.

5. The method according to claim 2, **characterised in that**, in the process of production of titanium dioxide nanoteeth on the surface of titanium alloy in pulse electrochemical oxidation of the implant surface, the distance between the cathode made of platinum and the anode being the modified surface of the implant ranges from 1.5 to 2.5 cm.

6. The method according to claim 5, **characterised in that**, after the anodic oxidation process, the implant is rinsed in an ultrasonic cleaner in distilled water with the addition of Al₂O₃ powder whose grain sizes are at the level of 50 nm, and then dried in a stream of argon.

7. The method according to claim 2, **characterised in that** the temperature of Ca(thd)₂ precursor evaporation is 188°C.

8. The method according to claim 2, **characterised in that** the temperature of CaCO₃ deposition is 250°C.

9. The method according to claim 2, **characterised in that** nitrogen is used as the carrier gas in the process of enrichment of the surface layer of the titanium alloy with hydroxyapatite nanograins.

10. The method according to claim 2, **characterised in that** the process of enrichment of the surface layer of the titanium alloy with hydroxyapatite nanograins lasts 100 or 500 cycles.
